# EUROPEAN PATENT APPLICATION

(11) **EP 4 843 545 A2**
(43) Date of publication of application: **25.11.2026**
(21) Application number: 26205289.7
(22) Date of filing: 13.12.2016
(51) Int. Cl.: A61F 9/007

(54) **OCULAR IMPLANT SYSTEMS**

(30) Priority: 24.12.2015 EP 15202724
(62) Document number(s) of the earlier application(s) in accordance with Art. 76 EPC: 22199340.5 / 4 137 102; 16820191.1 / 3 393 412
(71) Applicant: iSTAR Medical, 1300 Wavre (BE)
(72) Inventor: Vandiest, Nicolas, 1400 Nivelles (BE); De Marco, Emiliano, 4430 Ans (BE); Roy, Cécile, 5024 Marche-les-Dames (BE)
(74) Representative: De Clercq & Partners

(57) **Abstract**

Described herein is an ocular implant system in which an implant (200) is deployed in a posterior space of the eye, for example, the suprachoroidal space or the subconjunctival space or the intrascleral space, with an access within the anterior chamber (160) for reducing intraocular pressure therein. The shunt (200) is implanted using an implantation device comprising a hollow shaft (330) mounted over a fixed shaft (320). The shunt (200) is located in a distal end of the hollow shaft (330) and adjacent a distal end of the fixed shaft (320), the distal end of the fixed shaft being located at a position behind the distal end of the hollow shaft. Once the distal end of the hollow shaft (330) with the shunt (200) is located at the desired depth within the posterior space, the hollow shaft is withdrawn over the fixed shaft towards the proximal end thereof to leave the shunt in the posterior space with an access within the anterior chamber (160).

## Description

### Field of the invention

The present invention relates to ocular implant systems, and is more particularly, although not exclusively, concerned with devices for delivering ocular implants to a predetermined location in the eye.

### Background of the invention

The mammalian eye comprises an anterior chamber located between the cornea and the iris and lens. This chamber is filled with a fluid known as aqueous humour. A trabecular meshwork, comprising a plurality of microscopic passageways, is located in the angle between the iris and the cornea. In the normal human eye, aqueous humour is generated at a constant rate, typically about 2.2 to 2.7 microlitres per minute (µl/min), by the ciliary body behind the iris. In the conventional outflow pathway, this aqueous humour flows past between the lens and iris and then exits via the trabecular meshwork and is returned to the circulatory system.

The intraocular pressure (IOP) maintaining this outflow from the normal eye tends to remain within a range of 10 mmHg to 20 mmHg. However, there may be significant changes in the IOP related to the cardiac cycle, blinking, diurnally and other causes. In the most prevalent chronic form of glaucoma where the iridocorneal angle remains open, there is blockage of the trabecular meshwork fluid outflow path which causes a build-up of excess fluid in the eye, and, consequently raises IOP to a value consistently greater than about 18 mmHg. In some cases, the IOP may be as high as 50 mmHg or more. Over time, this pressure increase results in irreversible damage to the optic nerve and loss of vision.

Glaucoma is a major cause of blindness worldwide and affects over 80 million people. Glaucoma is associated with many conditions including high blood pressure, diabetes, steroid use and ethnic origins. Various treatments are currently available for glaucoma including drug regimes, laser trabeculoplasty, and trabeculectomy and intraocular drainage implants.

Drugs are frequently administered in the form of eye drops to control fluid inflow, that is, the formation of aqueous humour by the ciliary body, or to facilitate fluid egress through the trabecular meshwork. Erratic dosages, side effects and poor patient compliance are common issues.

In an alternative to drug use for glaucoma treatment, surgical creation of shunt paths or drains around or through the meshwork blockage is adopted as a means to release excess fluid and hence the build-up of IOP. In trabeculoplasty, a laser is used to create small openings in the trabecular meshwork of the eye so that aqueous humour can drain through the meshwork to reduce the intraocular pressure in the anterior chamber of the eye. This method of treatment is mainly used for open angle glaucoma.

Surgical techniques include trabeculectomy. Trabeculectomy is a surgical technique in which an small opening is created in the trabecular meshwork to allow the fluid to flow out of the anterior chamber, to collect under the conjunctiva, and be reabsorbed by the posterior parts of the eye.

Implanted devices are most often used where other treatment methods have become ineffective. These implants comprise drainage devices that are inserted into the eye so that aqueous humour can be drawn through a drainage path and away from the anterior chamber. With traditional implants, for example, Molteno implants, Baerveldt shunts, and Ahmed valves, a drain path is formed by means of a tube placed between the anterior chamber and a fluid dispersion plate located supra-sclerally, below the conjunctiva. Dispersed fluid from the plate forms a pool or "bleb" that is slowly re-absorbed into the outer layers of the eye. More recently, a new generation of drainage devices has been designed to be implanted through a minimally invasive approach, i.e. limited surgical manipulation of the conjunctiva and of the sclera, hence exhibiting a safer surgical profile than traditional drainage implants and altering the glaucoma treatment paradigm. Those micro-drainage devices, called MIGS (Minimally-Invasive Glaucoma Surgery), for example, iStent Supra^{®} Suprachoroidal Micro-Bypass Stent, Cypass Micro-stent and Xen Gel Stent, are designed to reduce intraocular pressure by accessing either the suprachoroidal space or the subconjunctival space.

In the iStent Supra^{®} Suprachoroidal Micro-Bypass Stent and Cypass Micro-stent, there is a lumen extending longitudinally through the stent, a guide or shaft being inserted into the lumen for positioning within targeted tissue, and when correctly positioned, the guide or shaft being retracted leaving the stent in the targeted tissue. One such implant is described, for example, in US-B-8337393.

US-B-8852136 discloses the implantation of intraocular shunts into the intrascleral space of the eye. The implantation device comprises a hollow shaft configured to hold an intraocular shunt, and is configured for insertion into the eye, and, from which the intraocular shunt is ejected or deployed before the hollow shaft is removed from the eye. The intraocular shunt is positioned so that it forms a passage from the anterior chamber to the intrascleral space of the eye so that aqueous humour can be drained from the anterior chamber into an episcleral vessel complex of the eye. The placement of the intraocular shunt also allows diffusion into the subconjunctival or suprachoroidal spaces.

US-B-8388568 also discloses implantation of an intraocular shunt into an eye. A deployment device is configured to hold an intraocular shunt as it is inserted into targeted tissue within the eye, and once in position within the targeted tissue, the intraocular shunt is deployed or ejected therefrom. In some embodiments of the deployment device, a portion thereof is designed to provide resistance indicating that the deployment device has been advanced across the anterior chamber and is correctly positioned for deployment of the intraocular shunt. The deployment of the intraocular shunt may provide for flow of aqueous humour from the anterior chamber into the subconjunctival or suprachoroidal space.

Whilst devices are known for implanting stents and intraocular implants, they tend to have disadvantages, such as, the stent needs to be mounted on a guide or shaft by means of a lumen for implantation or the implant needs to be ejected from the device into the correct location within the eye as described in US-B-8852136 and US-B-8388568 described above. In addition, such devices are inserted into the eye, the stent or intraocular implant deployed from the device and the device is removed from the eye.

WO-A-2011/084550 describes an implantation system for implanting substances into the retinal region of the eye. The implantation system comprises a delivery unit support or housing, a delivery unit (in the form of a mandrel or fixed shaft and a mandrel guide or hollow shaft) mounted within the delivery unit support or housing, and a delivery controller or slider mounted on the outside of the delivery unit support. The delivery controller may be attached either to the mandrel or fixed shaft or to the mandrel guide or hollow shaft with movement of the mandrel through the mandrel guide being considered to be an active movement and retraction of the mandrel guide over the mandrel considered to be a passive movement. A spring may be used to bias the delivery unit in a longitudinal direction for either active or passive movement. In one embodiment, the substance to be implanted is located at the distal end of the mandrel, and in other embodiment, a nozzle may be provided on the distal end of the mandrel guide for retaining the substance to be implanted.

However, in each embodiment of the implantation system described in WO-A-2011/084550, as the delivery controller is only attached to either the mandrel or the mandrel guide, there is no stability provided within the housing to ensure smooth and stable movement as the substance is implanted into the eye.

### Summary of the invention

It is therefore an object of the present invention to provide an implantation device which does not require that an implant is ejected from the device.

It is another object of the present invention to provide an implantation device in which the implant does not need to be mounted on a guide during implantation into the eye.

It is yet another object of the present invention to provide an ocular implant system in which the implantation device uses a simple forward and backward movement to deliver the implant in the eye.

It is a further object of the present invention to provide an ocular implant system in which the implantation device includes an implant in a portion thereof and the implant is left in a posterior space of an eye with a portion of the implant providing an access to the anterior chamber.

In accordance with one aspect of the present invention, there is provided an implantation device for implanting an implant into a posterior space in an eye, the device comprising:
a housing;
a fixed shaft having a proximal end and a distal end, the proximal end being mounted within the housing and fixed with respect thereto, the distal end thereof extending from the housing;
a delivery mechanism comprising a sliding element mounted on the fixed shaft and at least partially within the housing, the sliding element being configured to be moved between a first position and a second position with respect to the housing and to at least the fixed shaft; and
a hollow shaft having a distal end and a proximal end and being configured to be mounted over the distal end of the fixed shaft and to be connected to the sliding element at its proximal end, the hollow shaft being configured to retain an implant within a portion thereof at its distal end, the hollow shaft being configured to be retracted over the fixed shaft by movement of the sliding element from its first to second positions to release the implant from within distal end of the hollow shaft;
characterised in that the delivery mechanism further comprises a slider shaft mounted within the housing and configured to be parallel to the fixed shaft, the sliding element being mounted on both the slider shaft and the fixed shaft.

By having a slider shaft configured to be parallel to the fixed shaft with the sliding element being mounted on both shafts, smooth and stable movement of the sliding element is provided as the hollow shaft is retracted over the fixed shaft.

In addition, the implantation device of the present invention has the advantage that the delivery mechanism operates only on the hollow shaft to retract it over the fixed shaft leaving the implant in place within posterior space of the eye. In effect, no contact is necessary with the intraocular implant itself during its deployment from the distal end of the hollow shaft.

Moreover, there is no need to eject the implant from the implantation device or to inject the implant into the posterior space of the eye.

In one embodiment, the proximal end of the hollow shaft is mounted to a fixing element, the fixing element being configured for insertion into the housing over the fixed shaft and for engagement with the sliding element within the housing. The fixing element may comprises a connection portion configured to engage with a sliding element connection portion. In addition, the connection portion of the fixing element may comprise an indent providing at least two engagement surfaces, and, the sliding element connection portion comprises at least two end portions configured to be pushed apart by the insertion of the fixing element, the at least two end portions being configured to provide engagement surfaces which engage with the engagement surfaces of the fixing element.

In one embodiment, the fixing element comprises a body portion having a surface thereof configured to indicate a correct orientation for insertion of the fixing element into the housing.

In a preferred embodiment, the fixing element comprises a one-touch fixing connection element.

In one embodiment, the hollow shaft has an internal profile which is configured to match substantially the external profile of the fixed shaft. By having matching internal and external profiles of the hollow shaft and the fixed shaft, the hollow shaft can use the fixed shaft as a guide to ensure a smooth retraction thereover to release the implant from the distal end of the hollow shaft and leave it in place within the posterior space.

In one embodiment, the hollow shaft comprises a substantially transparent plastics material, the substantially transparent plastics material comprising one of: a thermosetting plastics material and a thermoplastics material. The plastics material may be flexible or rigid and may comprise a biocompatible plastics material. Alternatively, the hollow shaft comprises a biocompatible metal material. The use of biocompatible materials prevents irritation of the eye during the implantation process.

It is preferred that the hollow shaft have a bevelled tip. Preferably, the bevelled tip is configured to be atraumatic to reduce damage and irritation to the eye during the implantation process. It is also preferred that the distal end of the hollow shaft be configured to be flexible to match the curvature of ocular tissues of the eye where the implant is to be implanted.

In another embodiment, the hollow shaft comprises at least one marker indicating an insertion depth for the posterior space with respect to an anterior chamber of the eye. By using one or more markers on the hollow shaft, one or more indications are provided to ensure that the implant is correctly positioned within the posterior space.

In a further embodiment, the implantation device comprises a light source configured to provide light for visual contrast between the implant and the posterior space of the eye. At least one light-emitting source may be provided which is configured to emit at least one colour of light. The provision of a light source has the advantage that the implant becomes more visible and is readily discernible within the eye. Selection of the colour of the light enables correct positioning of the implant as it can be a colour which is either absorbed or reflected by the implant so that there is a definite contrast between the surrounding tissue. It may be the case that the colour of light is chosen in accordance with the surrounding tissue to ensure that there is a contrast.

An optical waveguide may be provided which is connected to the light source, the optical waveguide being configured for directing light emitted by the light source to the distal end of the hollow shaft. By using an optical waveguide, the light may be directed more appropriately so that light does not flood the eye during implantation of the implant. The optical waveguide may be formed on either at least one part of the fixed shaft or on at least one part of the hollow shaft. This means that no additional components are required for directing the light towards the distal end of the hollow shaft.

In manual operation of the implantation device, a button is mounted on the housing and is configured to be moved with respect to the housing, the button being connected to the delivery mechanism and is configured to move the sliding element between its first and second positions when moved with respect to the housing. In effect, movement of the button in a direction from the distal end to the proximal end of the fixed shaft causes the retraction of the hollow shaft over the fixed shaft to release the implant from the hollow shaft.

In accordance with another aspect of the present invention, there is provided an ocular implant system configured for implanting an implant into a posterior space in an eye, the ocular system comprising: an implant configured for being implanted in the posterior space in the eye; and an implantation device as described above, the implant being located in the distal end of the hollow shaft.

In one embodiment, the ocular implant system may be provided for "single-use" and is assembled ready for implanting the implant into the posterior space of the eye. This has the advantage that there is no need to position the implant within the hollow shaft or to mount a hollow shaft, with implant located within its distal end, on the fixed shaft each time an implant is to be implanted.

In another embodiment, an actuating mechanism configured to be flexibly connected to the implantation device and being configured to move the sliding element of the delivery mechanism between its first and second positions. By having a separate actuating mechanism which does not form part of the implantation device, better control can be provided for the retraction of the hollow shaft over the fixed shaft. In effect, a smooth retraction can be provided as the actuating mechanism can be operated using a different hand to that in which the implantation device is held.

In a further embodiment, the actuating mechanism comprises a pneumatic plunger device configured to be connected to an operating mechanism, the operating mechanism being connected to the delivery mechanism and comprising an inner portion and an outer portion at least partially surrounding the inner portion, the inner and outer portions being configured to be moveable relative to one another. By having an operating mechanism which comprises an inner portion and an outer portion and which connects the actuating mechanism to the implantation device, one portion can determine the spacing between the two devices whilst the relative movement of the other portion can activate the sliding element of the implantation device. In a preferred embodiment, the operating mechanism comprises an inner wire connecting the sliding element in the delivery mechanism to the separate actuating mechanism over which an outer sheath is provided. The inner wire is slidable within the outer sheath.

The implant may comprise at least one marker configured to indicate an insertion depth of the implant within the posterior space with respect to the anterior chamber and the eye. This has the advantage of providing a way of determining if the implant is correctly positioned within the posterior space. It is preferred that the marker comprises a colour chosen to provide contrast within the anterior chamber.

In accordance with another aspect of the present invention, there is provided a housing for an implantation device including: a fixed shaft having a proximal end and a distal end, the proximal end being mounted within the housing and fixed with respect thereto, the distal end thereof extending from the housing; and a delivery mechanism comprising a sliding element mounted on the fixed shaft and at least partially within the housing, the sliding element being configured to be moved between a first position and a second position with respect to the housing and to at least the fixed shaft; characterised in that the delivery mechanism further comprises a slider shaft mounted within the housing and configured to be parallel to the fixed shaft, the sliding element being mounted on both the slider shaft and the fixed shaft.

In accordance with a further aspect of the present invention, there is provided a hollow shaft assembly for an implantation device including: a hollow shaft having a distal end and a proximal end and being configured to be mounted over the distal end of the fixed shaft and to be connected to the sliding element at its proximal end, the hollow shaft being configured to retain an implant within a portion thereof at its distal end, the hollow shaft being configured to be retracted over the fixed shaft by movement of the sliding element from its first to second positions to release the implant from within distal end of the hollow shaft; an implant positioned in the distal end of the hollow shaft; and a fixing connection element to which the hollow shaft is connected.

In accordance with yet a further aspect of the present invention, there is provided an ocular implantation kit comprising a housing and a hollow shaft assemby as described above.

### Brief description of the drawings

For a better understanding of the present invention, reference will now be made, by way of example, to the accompanying drawings in which:-
Figure 1 illustrates a sagittal sectioned view of an eye;
Figure 2 illustrates an enlarged cross-sectioned view of the eye showing portions of the anterior chamber and outer layers of the eye;
Figure 3 illustrates an implantation device in accordance with the present invention;
Figure 4 illustrates the interior of the implantation device of Figure 3;
Figure 5 illustrates an exploded view of the implantation device of Figure 3;
Figure 6 illustrates a hollow shaft forming part of the implantation device of Figure 3 together with an implant to be implanted;
Figure 7 illustrates an enlarged view of the implant shown in Figure 6;
Figure 8 illustrates two alternative configurations for the implant to be used in the implantation device of Figure 3;
Figure 9 illustrates implantation of the implant using the minimally invasive *ab interno* method;
Figure 10 illustrates the removal of the implantation device;
Figure 11 illustrates the implant in position in the eye;
Figure 12 illustrates an actuation mechanism connected to the implantation device of Figure 3;
Figure 13 illustrates the actuation mechanism of Figure 12 with one part of its handle removed;
Figure 14 illustrates an enlarged view of a part of the actuation mechanism of Figures 12 and 13;
Figure 15 illustrates an exploded view of the actuation mechanism of Figures 12 to 14;
Figure 16 illustrates a perspective view of another embodiment of an implantation device in accordance with the present invention;
Figure 17 illustrates a perspective view of the implantation device of Figure 16 with a portion of the handle removed;
Figure 18 illustrates a perspective view of a sliding element implemented in the embodiment shown in Figures 16 and 17;
Figure 19 illustrates a first embodiment of a one-touch fitting connection with a hollow shaft; and
Figure 20 illustrates a second embodiment of a one-touch fitting connection with a hollow shaft.

### Description of the invention

The present invention will be described with respect to particular embodiments and with reference to certain drawings but the invention is not limited thereto. The drawings described are only schematic and are nonlimiting. In the drawings, the size of some of the elements may be exaggerated and not drawn on scale for illustrative purposes.

The present invention relates to a system which comprises a single-use "minimally-invasive" implantation or deployment device from which an intraocular shunt or implant is deployed into the suprachoroidal space, that is, the space lying between the sclera and the choroid of the eye, or into the subconjunctival space, that is, the space lying between the conjunctiva and the sclera of the eye. The intraocular shunt or implant is pre-loaded within a portion of the implantation or deployment device and is released therefrom as will be described in more detail below. In one embodiment, the implantation or deployment device is single use, but it will readily be appreciated that the implantation or deployment device may be re-useable.

The intraocular shunt or implant provides a mechanism for providing a drainage pathway between the anterior chamber of the eye to a posterior part of the eye (suprachoroidal space, intrascleral space or subconjunctival space) as will be described below with reference to the drawings. The intraocular shunt or implant may be made from a biocompatible material such as that described in EP-B-2517619. It will readily be appreciated that the intraocular shunt or implant may be made from other suitable biocompatible materials, for example, silicone.

In EP-B-2517619, the described biocompatible material is porous and comprises a biocompatible polymer scaffold that defines an array of interconnected pores having similar diameters. Typically, the mean diameter of the pores is between about 20µm and about 90µm, preferably between about 25µm and about 75µm. For use in the implant of the present invention, the preferred range is between about 25µm and about 36µm.

The intraocular shunt or implant may be substantially cylindrical in shape with a rectangular or elliptical cross-section and having a length of 5mm, a width of 1.1mm and a depth of 0.6mm or may be substantially cylindrical with a circular cross-section and having a similar length with a particular diameter. The intraocular shunt or implant is punched out of a block or sheet of biocompatible material where the thickness of the block or sheet has a thickness which is equal to or greater than the length of the shunt or implant. It will readily be understood that the intraocular shunt or implant may be formed in other ways, for example, but using suitable cutting means other than punches.

The terms "targeted tissue" or "targeted tissues" as used herein refer to the tissue into which the intraocular shunt or implant is to be positioned, that is, the suprachoroidal space, the subconjunctival space or the intrascleral space.

The term "natural anchor" as used herein refers to a component which does not require any elements for holding it in the correct position once delivered or deployed within the eye.

The term "posterior" as used herein refers to a location behind the anterior chamber of an eye. The term may apply to a space which is present between tissue layers in the eye, for example, the suprachoroidal space and the subconjunctival space, or to a space which is created within one tissue layer in the eye, for example, the intrascleral space.

The terms "deploy" and "deployment" as used herein refer to the delivery of an intraocular shunt or implant from an implantation or deployment device into the targeted tissue within the eye. These terms are not intended to include injection, ejection or advancement of the intraocular shunt or implant from an implantation or deployment device where force is used to deliver the shunt or implant into the targeted tissue.

The terms "intraocular shunt", "shunt", "intraocular implant" or "implant" as used herein refer to a component which is implanted into the eye. The term "implant" is more general as it refers to anything that is implanted but is used to refer also to a shunt. The term "shunt" refers to a component through which aqueous humour can pass from the anterior chamber of the eye into the targeted tissue located therebehind.

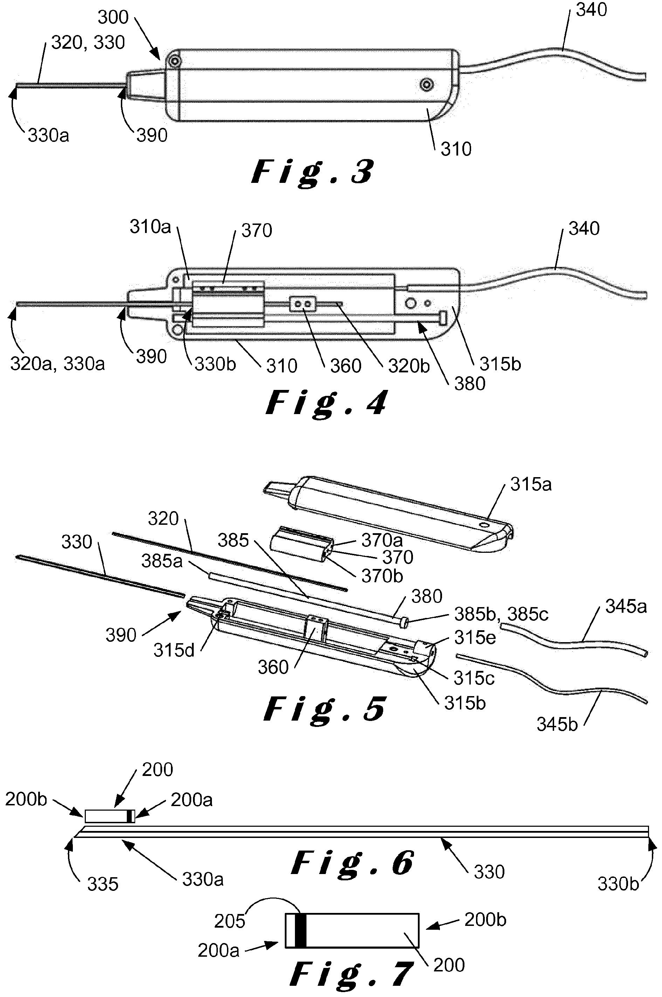

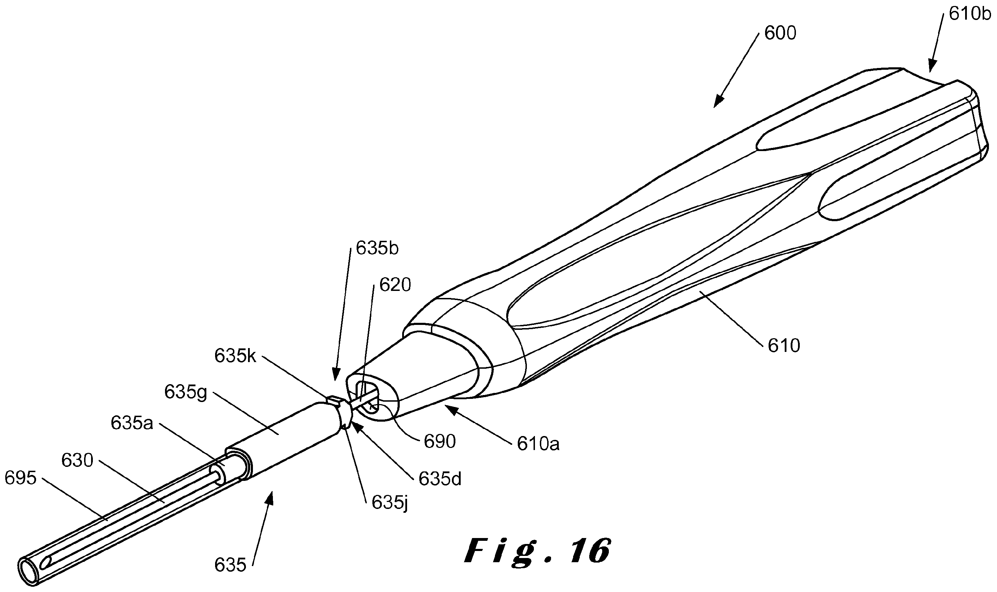

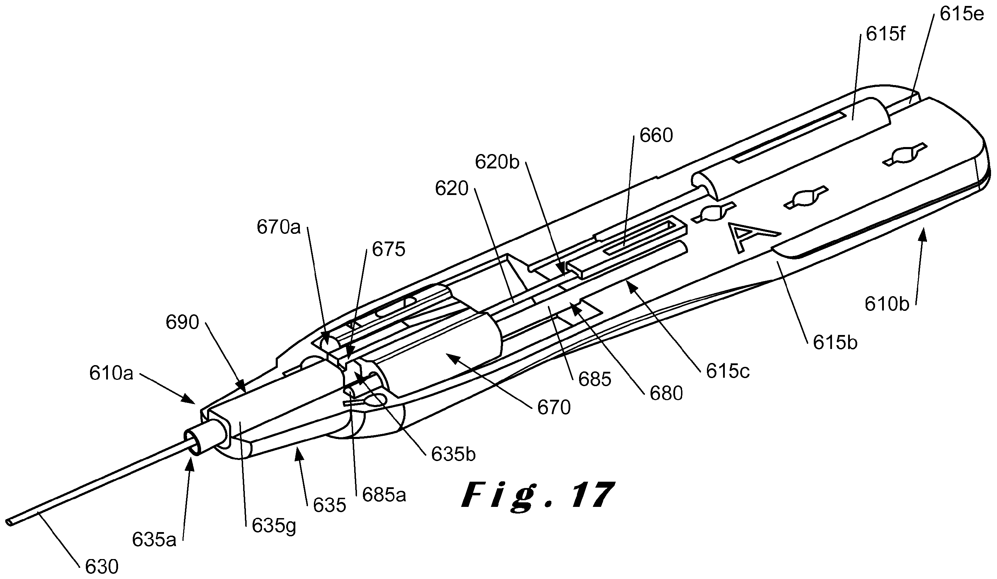

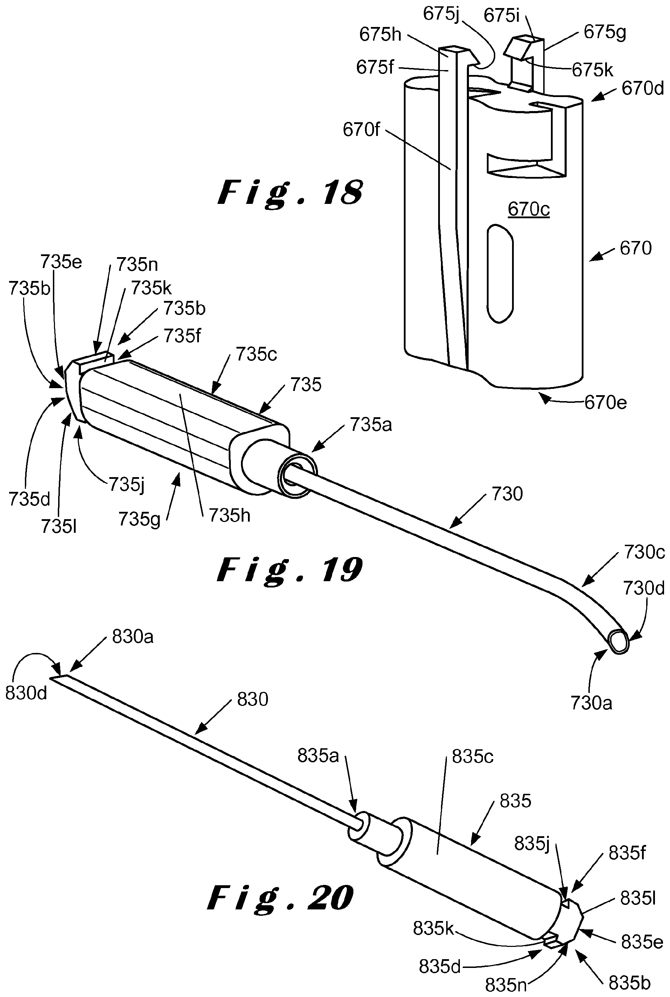

Components which are the same are referenced the same and those which are modifications and/or alternatives are either referenced with a suffix "A", "B" and the like, or with a prime ('), double prime (") and the like. In addition, components which are similar have the same last two digits with a different first digit, for example, the hollow shaft has the reference numeral 330 in the embodiments shown in Figures 3 to 6, the reference numeral 630 in Figures 16 and 17, the reference numeral 730 in Figure 19 and the reference numeral 830 in Figure 20.

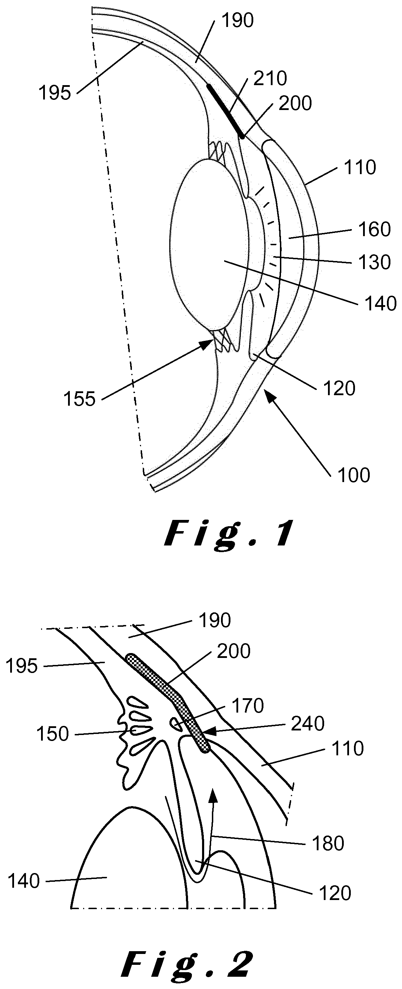

Referring initially to Figures 1 and 2, a sagittal section through an eye 100 is shown illustrating the position of the cornea 110, the iris 120, the pupil 130, the lens 140, and the ciliary body 150 (shown more clearly in Figure 2). The anterior chamber 160 is located between the lens 140 and the cornea 110.

In the normal eye, aqueous humour originates in the ciliary body 150 and, for the conventional outflow path, circulates between the iris 120 and lens 140 into the anterior chamber 160, and then exits via the porous trabecular meshwork 170 located in the iridocorneal angle 240 between the iris 120 and cornea 110 as indicated by arrow 180. The sclera 190 and the choroid 195 are also shown.

In the glaucomatous eye, meshwork 170 is commonly blocked, causing a damaging pressure increase inside the eye. An intraocular shunt or implant 200 in accordance with the present invention may be implanted between the sclera 190 and the choroid 195, that is, in the suprachoroidal space, with an access via the anterior chamber 160, to form a fluid path from the anterior chamber 160 to a sub-scleral area 210, bypassing this blockage and restoring the fluid flow.

The shunt or implant may be pre-loaded and compressed in a hollow shaft forming part of a deployment or implantation device. As the shunt or implant is compressed prior to deployment, it will expand, after deployment, once located within the targeted tissue, that is, either between the sclera and the choroid (suprachoroidal space) or between the conjunctiva and the sclera ( subconjunctival space) or within the sclera (intrascleral space), and integrate with the surrounding tissue to form a natural anchor therewith.

The shunt or implant may have markers provided thereon so that an operator deploying the shunt or implant can control the depth of the shunt or implant in the targeted tissue with a portion thereof remaining in the anterior chamber. Placement of the shunt or implant within the eye is made using an implantation or deployment device as will be described in more detail below.

The implantation device comprises a minimally invasive device for implanting an intraocular shunt or implant into the posterior space of the eye with an access via the anterior chamber. In one embodiment, the *ab interno* method, the hollow shaft containing the pre-loaded shunt or implant is inserted through the cornea, across the anterior chamber and into the targeted tissue. In a second embodiment, the *ab externo* method, the hollow shaft containing the pre-loaded shunt or implant is inserted from the targeted tissue into the anterior chamber. The implantation device of the present invention may be used for both *ab interno* and *ab externo* methods.

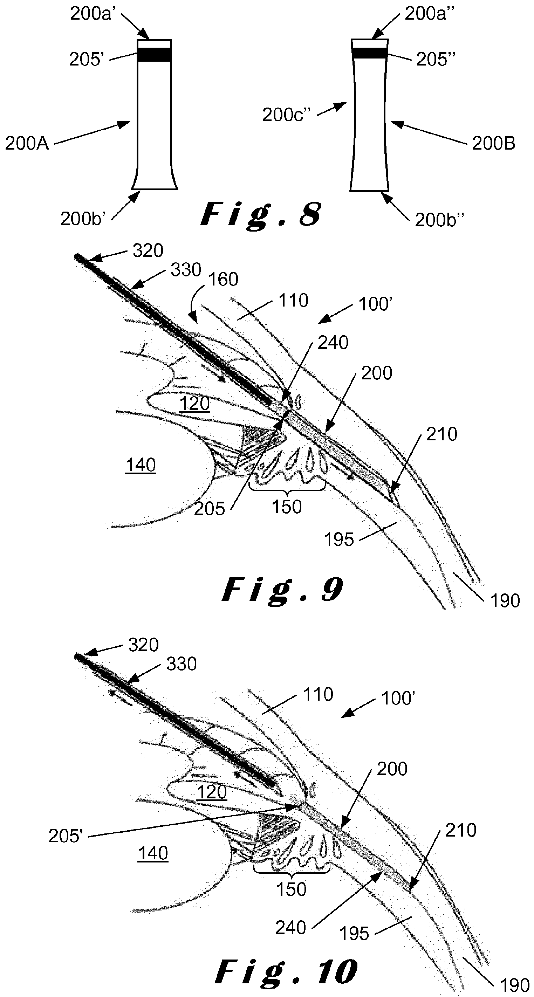

The implantation device, in its simplest form, comprises a manually-operated hand-held device configured for inserting a portion thereof through a corneal incision which may be made by a sharp portion of a distal end of the hollow shaft. The deployment of the shunt or implant is in accordance with the operation of an insertion and deployment mechanism described in more detail below with reference to the Figures 9 to 11.

In one embodiment of the implantation device, an ergonomically shaped handle or housing is provided having a proximal end and a distal end. A delivery mechanism including a linear slider is mounted in the handle or housing for movement in a direction defined between the distal end towards the proximal of the handle or housing. The delivery mechanism is attached to the handle with a portion thereof mounted within the handle or housing, the delivery mechanism comprising a shaft mounted within the handle or housing and is fixed relative thereto. The shaft has a proximal end within the handle or housing and a distal end extending from the handle or housing. A hollow shaft is mountable over the fixed shaft and has a proximal end engageable with the linear slider and a distal end in which the shunt or implant is pre-loaded. The hollow shaft may be made of any suitable biocompatible and sterilisable thermosetting or thermoplastic material, or alternatively, a biocompatible and sterilisable metal.

The hollow shaft is sized to fit around the fixed shaft and to be moveable relative to both the handle or housing and the fixed shaft as will be described in more detail below. The hollow shaft has an internal profile, and, the fixed shaft has an external profile, the internal profile of the hollow shaft being configured to match substantially the external profile of the fixed shaft. Such internal and external profiles may have any suitable cross-sectional shape, for example, circular, elliptical, rectangular, square, etc.

It will be appreciated that the hollow shaft also has an external profile which may be similar to or different from the internal profile thereof.

In an alternative embodiment of the device, an actuating mechanism is provided which is connectable to the delivery mechanism within the handle or housing by means of a flexible wire. The actuating mechanism comprising a plunger which, when activated, operates to retract the hollow shaft over the fixed shaft to deploy, release or leave the shunt or implant in the targeted tissues.

In a further embodiment, the plunger of the actuating mechanism may be pneumatically or electrically controlled so as to ensure a smooth operation thereof. In yet a further embodiment, the plunger of the actuating mechanism may form part of a controlled friction system including O-rings or springs for a smooth movement and/or translational speed control.

The implantation device is configured to extend the distal end of the hollow shaft into the targeted tissue space and to retract it therefrom leaving the shunt or implant in position within it. The linear slider in the handle retracts the hollow shaft over the fixed shaft to leave the shunt or implant correctly positioned in the targeted tissue without the need for injecting the shunt or implant into the targeted tissue.

The use of a separate actuating mechanism which is connected to the linear slider in the handle and activatable by a third party enables a smooth retraction of the hollow shaft while minimizing movements of the hand of the operator holding the implantation device.

In accordance with the present invention, there is no physical deployment of the shunt or implant into the eye by the fixed shaft as it only operates to maintain the shunt or implant in place while retracting the hollow shaft.

The hollow shaft may have an internal profile of circular cross-section or any other suitable cross-section into which the shunt or implant is accommodated as described above. In one embodiment, the shunt or implant may have a similar cross-section to that of the internal profile of the hollow shaft at least at the distal end thereof.

The distal end of the hollow shaft in which the shunt or implant is located for deployment may have the same or a different cross-section to the rest of the hollow shaft. The distal end of the hollow shaft may be flat or curved. The tip of the distal end may be bevelled or have a flat or tapered surface. The tip of the distal end of the hollow shaft may be blunt or sharp - if sharp, the tip may be used to make incisions through tissue in the eye to enable the shunt or implant to be correctly positioned in a posterior space of the eye. In addition, the hollow shaft may have markers located thereon to indicate the positioning of the shunt or implant within the distal end thereof. In one embodiment, at least the distal end of the hollow shaft could be transparent.

The handle or housing may further include an embedded light-emitting diode (LED) illumination source which is coupled to the hollow shaft via a fibre optic cable or an optical waveguide. In one embodiment, the hollow shaft may be configured so that at least at its distal end is transparent so that the light can be directed towards the intraocular shunt or implant to be delivered to the targeted tissue. The optical waveguide may be formed in a part of either one of the hollow shaft or the fixed shaft.

The LED illumination source may comprise at least one LED element which may emit at least one colour of light. It may be possible that the colour of light emitted by the at least one LED element may be selectable in order to provide a visual contrast which enables the location of the shunt or implant to be determined in targeted tissue.

Turning now to Figure 3, an implantation device 300 is shown which comprises a handle or housing 310 and a fixed shaft 320 mounted within the handle or housing such that a distal end 320a thereof extends from the handle or housing with the proximal end 320b being located within the handle or housing 310 (see Figure 4). A hollow shaft 330 is mounted on the fixed shaft 320 with a proximal end thereof (not shown) mounted within the handle or housing 310 and a distal end thereof 330a extending over the distal end 320a of the fixed shaft 320. A wire 340 is shown which connects the implantation device to an actuating mechanism as will be described in more detail below.

Figure 4 illustrates the implantation device 300 with a first portion 315a of the handle or housing 310 removed. As shown, the proximal end 320b of the fixed shaft 320 is connected to a second portion 315b of the handle or housing 310 by a fixing element 360. The fixed shaft 320 and a slider shaft 380 pass through a sliding element 370 which is configured to be slideable between a first position where it abuts an end face 310a of the handle or housing 310 and a second position where it abuts the fixing element 360.

The proximal end 330b of the hollow shaft 330 engages with the sliding element 370 and is configured to slide over the fixed shaft 320 when the sliding element 370 is moved from the first position to the second position.

An exploded view of the implantation device 300 is shown in Figure 5. As shown, the handle or housing 310 comprises a first portion 315a and a second portion 315b. As described above, the fixed shaft 320 is mounted within the handle or housing 310 by the fixing element 360, the fixing element 360 being fixed to the second portion 315b of the handle or housing 310.

Parallel to the fixed shaft 320 is located the slider shaft 380. The slider shaft 380 may comprise a substantially cylindrical portion 385 on which the sliding element 370 can slide from a distal end 385a to a portion adjacent the fixing element 360. At a proximal end 385b of the slider shaft 380, a head portion 385c may be provided which is retained within a slot 315c of the second portion 315b. Similarly, the distal end 385a of the slider shaft 380 is located within a slot 315d of the second portion 315b. Although shown as separate slots in Figure 5, it will be appreciated that slots 315c, 315d may comprise a single shaped slot extending within the second portion 315b to accommodate the ends 385a, 385b, 385c of the slider shaft 380.

The wire 340 comprises an external flexible hollow wire 345a and a internal rigid wire 345b. The external wire 345a is connected to a slot 315e of the second portion 315b of the handle or housing 310 and the internal wire 345b is connected to the sliding element 370 through a hole 370a and is configured to actuate movement thereof with respect to the handle or housing 310, that is, the internal wire 345b operates to move the sliding element 370 towards the proximal end 310b of the handle or housing 310. By moving the sliding element 370 towards the proximal end 310b of the handle or housing, the hollow shaft 330 is retracted over the fixed shaft 320 leaving the shunt or implant 200 in place within the posterior part of the eye as will be described in more detail below.

When the first portion 315a of the handle or housing 310 is mounted on the second portion 315b thereof, the fixed shaft 320 attached to the fixing element 360, the sliding element 370 and the slider shaft 380 are retained therein.

The distal end 310a of the handle or housing 310 has an elongated aperture 390 formed therein through which the distal end 320a of the fixed shaft 320 protrudes. The proximal end 330b of the hollow shaft 330 is configured to be inserted through the aperture 390 and to engage with a portion of the sliding element 370 (not shown).

The hollow shaft 330 is shown in Figure 6 together with a shunt or implant 200 which is also shown in Figure 7. In one embodiment, the hollow shaft 330 may have a circular cross-section with a circular internal cross-section. In another embodiment, the hollow shaft 330 may have an oblong cross-section, that is, a cross-section having two substantially parallel edges joined by an arc at each end. In a further embodiment, the hollow shaft 330 may have a rectangular cross-section with a rectangular internal cross section. In other embodiments, the cross-section may be such that the internal cross-section is a different shape to cross-section defined by the external shape of the hollow shaft 330.

The shunt or implant 200 may have a marker 205 located in the proximity of its proximal end 200a so that it is possible to see its position through a substantially transparent hollow shaft 330. It will readily be appreciated that other markers may be provided on the shunt or implant 200 for better visibility, for example, two markers may be provided at the proximal end 200b of the shunt or implant 200.

As described above, the shunt or implant 200 is located in the distal end 330a of the hollow shaft 330 prior to implantation and comprises a suitable biocompatible material as described above. Typically, the shunt or implant may be between 4mm and 7mm long and may have a diameter between 0.4mm and 2mm. In one embodiment, the shunt or implant 200 has a circular cross-section, but it will readily be appreciated that it may have any suitable cross-section which matches the internal cross-section of the hollow shaft 330. In another embodiment, the shunt may have a rectangular cross-section with a length between 3mm and 9mm, a thickness between 0.3mm and 1mm and a width between 0.5mm and 2mm. In a preferred embodiment, the shunt is 5mm long, 0.6mm thick and 1.1 mm wide. In a further embodiment, the shunt may have an elliptical cross-section with similar dimensions to that of the shunt having a rectangular cross-section.

Figures 8a and 8b illustrate two alternative configurations to that shown for the shunt or implant 200. In Figure 8a, the shunt or implant 200A is flared at its distal end 200b' with a marker 205' at its proximal end 200a'; and in Figure 8b, the shunt or implant 200B is flared at both its distal end 200b" and its proximal end 200a" with a "waist" portion 200c" therebetween. As before, a marker 205" is provided at its proximal end 200a".

In one embodiment, the implantation device 300 is provided as a sterilised unit which is ready for use when its sterile packaging has been removed, that is, the handle or housing 310 (with its internal components as described above with reference to Figures 3 to 5), the fixed shaft 320 and the hollow shaft 330 including the shunt or implant 200 inside its distal end 330a.

When assembled, the portion of the fixed shaft 320 extending from the sliding element 370 is shorter than the portion of the hollow shaft 330 extending from the sliding element 370 by at least the length of the shunt or implant 200. This means that the shunt or implant 200 is located in the distal end 330a of the hollow shaft 330 against an end of the fixed shaft 320 at its distal end 320a in the implantation configuration.

Although not shown, the sliding element 370 may be biased to the first position by a resilient element located on the fixed shaft 320 between the sliding element 370 and the fixing element 360, and movement to the second position is against the action of the resilient element to ensure a smooth movement of the hollow shaft 330 over the fixed shaft 320 during release of the intraocular shunt or implant. It will be appreciated that the resilient element may be located at any other suitable location within the handle or housing to provide appropriate biasing of the sliding element 370. The resilient element may be a compression spring or any other suitable resilient element which is deformable when the sliding element 370 is moved from its first to its second position in the handle or housing.

Figure 9 illustrates the implantation of an intraocular shunt or implant 200 using the *ab interno* method. As shown, the shunt or implant 200 is retained within the hollow shaft 330 which is mounted on the fixed shaft 320 - the full length of the hollow shaft 330 and the fixed shaft 320 not being shown for clarity. In this embodiment, the hollow shaft 330 has a bevelled tip 335 at its distal end 330a, the bevelled tip being used to ease penetration of the hollow shaft through the cornea 110. The hollow shaft 330 is then directed into and across the anterior chamber 160 of the eye to the iridocorneal angle 240 and into the sub-scleral space 210. As shown, the bevelled tip 335 at the distal end 330a of the hollow shaft 330 is also configured to provide an soft penetration into the subscleral space as shown.

It will readily be appreciated that the distal end 330a of the hollow shaft 330 does not need to provide the incision into the cornea and this can be done by a separate tool with the hollow shaft being inserted into the incision made.

Figure 10 is similar to Figure 9 but illustrates the intraocular shunt or implant 200 in position within the subscleral space 210. The hollow shaft 330 is shown retracted with respect to the fixed shaft 320. During the deployment of the intraocular shunt or implant 200 within the subscleral space after it has been correctly positioned, the sliding element 370 is moved within the handle or housing 310 from the distal end 310a thereof towards the fixing element 360. This movement causes the hollow shaft 330, which is connected to the sliding element 370, to be retracted over the fixed shaft 320 leaving the shunt or implant 200 in position within the subscleral space.

In the simplest embodiment, a portion of the sliding element 370 may extend through portion 315a of the handle or housing 310 and is configured to be manually moved by the medical practitioner implanting the shunt or implant 200 in the eye from its first position to its second position. However, in other embodiments, the sliding element 370 is located in the handle or housing 310 and is configured to be operated using an actuating mechanism as will be described in more detail below. It is important to have smooth and controlled retraction of the hollow shaft 330 so that the shunt or implant 200 is not moved or damaged during the withdrawal of the hollow shaft 330 of the implantation device 300 from the iridocorneal angle 240 and that there is no damage to the eye itself.

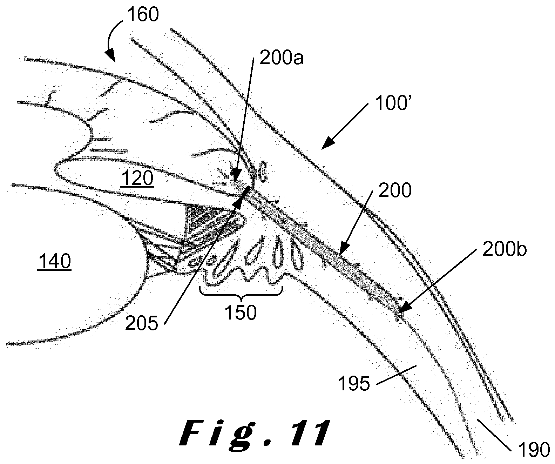

Figure 11 illustrates the shunt or implant 200 in position with the implantation device removed from the eye. As shown, the majority of the shunt or implant 200 is positioned in the subscleral space 210 with the proximal end 200a thereof in the anterior chamber 160. Aqueous humour flows from the anterior chamber 160 to the suprachoroidal space, hence reducing the ocular pressure of the eye.

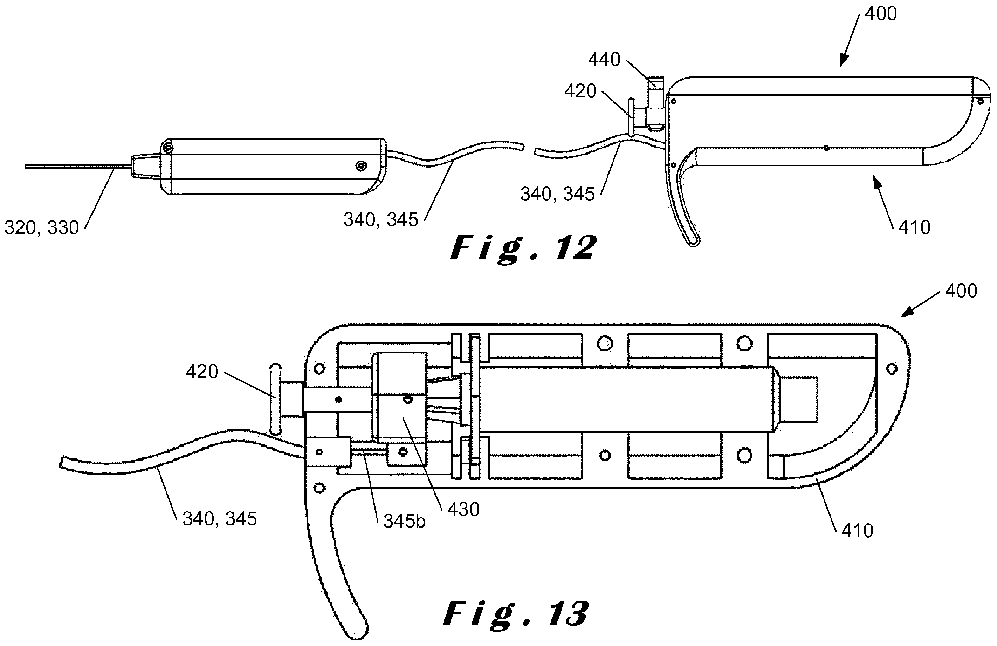

As described above with reference to Figures 3 to 5, the implantation device 300 also includes a wire 340 comprising an external flexible hollow wire 345a and an internal rigid wire 345b. Figures 12 to 14 illustrate the connection of the implantation device 300 to an actuating mechanism 400 via wire 340.

The actuating mechanism 400 comprises a handle 410 in which a plunger 420 is mounted, the actuating mechanism being connected to the handle 410 via the wire 340. The external wire 345a is connected to a slot 410a of the actuating mechanism 400 and the internal wire 345b is connected to a portion 430 of the plunger 420. Depression of the plunger 420, that is, movement of the plunger 420 towards the handle 410, causes the internal wire 345b to be drawn out of the handle 310 of the implantation device 300 moving the sliding element 370 from its first position to its second position and retracting the hollow shaft 330 over the fixed shaft 320. In order to prevent premature depression of the plunger 420, a stop element 440 (Figure 12) is provided for preventing movement of the plunger until it is removed.

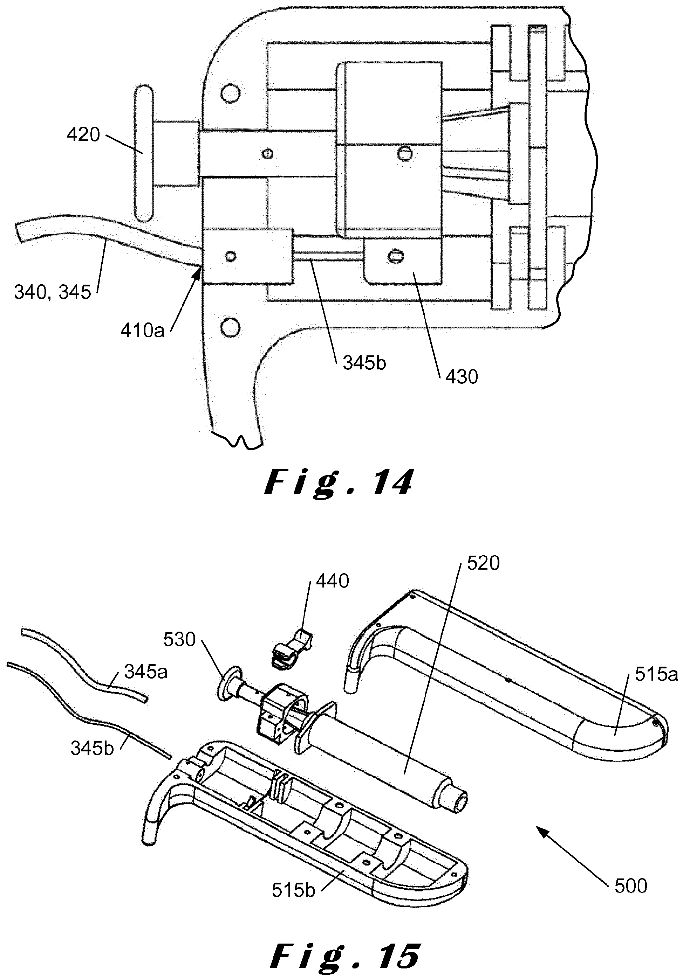

In Figure 15, a pneumatic actuating mechanism 500 is shown which comprises a housing or handle which is similar to housing or handle 410. The housing comprises a first portion 515a and a second portion 515b which a piston (shown generally as 520) is mounted. A plunger 530 is connected to the piston 520, and, when the plunger 530 is depressed, the piston is operated and the internal wire 345b is drawn is out of the handle 310 of the implantation device 300 to retract the hollow shaft 330 over the fixed shaft 320 as described above. Stop element 440 is also shown and operates as described above with reference to Figure 12.

It will readily be understood that smooth and controlled operation of the actuating mechanism is desired, and, by having the plunger of the actuating mechanism pneumatically controlled smooth and controlled operation thereof can be ensured. As an alternative, the plunger of the actuating mechanism may form part of a controlled friction system including O-rings or springs for a smooth movement and/or translational speed control (not shown).

Figure 16 illustrates a further embodiment of an implantation device 600 which comprises a handle or housing 610 and a fixed shaft 620 mounted within the handle or housing 610. As shown, the handle or housing 610 has a distal end 610a and a proximal end 610b.

The device 600 also includes a hollow shaft assembly comprising a hollow shaft 630 attached to a one-touch fitting connection element (OTFC) 635. The OTFC 635 is hollow so that the fixed shaft 620 can pass therethrough and into the hollow shaft 630 when the OTFC 635 is mounted within the distal end 610a of the handle or housing 610.

Distal end 635a of the OTFC 635 retains the hollow shaft 630 and proximal end 635b of the OTFC 630 is configured to be mounted within a sliding element 670 (as will be described in more detail with reference to Figure 19) when inserted into elongated aperture 690 formed in the distal end of the handle 610.

A removable cover 695 is provided over the distal end 635a of the OTFC 635 and over the hollow shaft 630. The cover 695 is intended to protect the hollow shaft 630, and the shunt or implant (not shown) located within the distal end 630a of the hollow shaft 630, as the OTFC 635 is inserted into in the handle 610. The removable cover 695 is optional and must be removed from the OTFC 635 after proximal end hollow shaft 630b has been coupled to the sliding element 670 in the handle or housing 610 and prior to the distal end 630a of the hollow shaft 630 being introduced into the eye during implantation of the shunt or implant.

Turning now to Figure 17, the implantation device 600 with a first portion (not shown) of the handle 610 removed to show the interconnection between the OTFC 635 and the sliding element 670. Proximal end 620a of the fixed shaft 620 is connected to a second portion 615b of the handle or housing 610 by a fixing element 660. The fixed shaft 620 and a slider shaft 680 pass through a sliding element 670 which is configured to be slideable between a first position where it abuts an end face 610a of the handle or housing 610 and a second position where it abuts the fixing element 660. The sliding element 670 is described in more detail below with reference to Figure 18.

Proximal end 635b of the OTFC 635 engages with connecting elements 675 of the sliding element 670 locating the hollow shaft 630 over the fixed shaft 620. As the hollow shaft 630 is fixed to the OTFC 635, and the proximal end 635b of the OTFC 630 engages with connecting elements 675 of the sliding element 670, the hollow shaft 630 is then fixed with respect to the sliding element 670 and is configured to slide over the fixed shaft 620 when the sliding element 670 is moved from the first position to the second position.

Slot 615e and connector 615f are shown which receive a wire or connection (not shown) from an external actuating mechanism (also not shown).

As shown in Figure 17, the fixed shaft 620 is mounted within the handle or housing 610 by the fixing element 660, the fixing element 660 being fixed to the second portion 615b of the handle or housing 610. Parallel to the fixed shaft 620 is located the slider shaft 680 in slot 615c. The slider shaft 680 may comprise a substantially cylindrical portion 685 on which the sliding element 670 can slide from a distal end thereof (not shown) to a portion adjacent the fixing element 660.

As described above with reference to Figures 3 to 5, by moving the sliding element 670 towards the proximal end 610b of the handle or housing 610, the hollow shaft 630 is retracted over the fixed shaft 620 leaving the shunt or implant 200, 200A, 200B (Figures 7 and 8) in place within the posterior part of the eye as described above.

When the first portion of the handle or housing 610 is mounted on the second portion 615b thereof, the fixed shaft 620 attached to the fixing element 660, the sliding element 670 and the slider shaft 680 are retained therein.

The distal end 610a of the handle or housing 610 has an elongated aperture 690 formed therein through which the distal end of the fixed shaft 620 protrudes (not shown). The proximal end 635b of the OTFC 635 is configured to be inserted through the aperture 690 and to engage with the connection elements 675 of the sliding element 670. Operation of the sliding element 670 is as described above with respect to Figures 3 to 5 and 13 to 15 above.

It will readily be appreciated that, although not clearly shown in Figures 16 and 17, the OTFC 630 has a generally rounded body portion 635g with a flat surface provided for the correct orientation when the OTFC 635 is inserted into the handle or housing 610. In addition, it will be readily understood that the hollow shaft 630 extends through the body portion 635g and terminates substantially at the proximal end 735b of the OTFC 735 to allow the fixed shaft 620 to be inserted therein as described above.

Figure 18 illustrates a sliding element 670 in more detail. The sliding element 670 comprises a body portion 670c having a distal end 670d and a proximal end 670e. As described above, the sliding element 670 has a longitudinally extending hole (not shown in Figure 18 but analogous to that shown in Figure 5 with respect to sliding element 370) through which the fixed shaft 630 extends into and through the elongate aperture 690 of the handle or housing 610 (as shown in Figure 16). Another longitudinally extending hole (also not shown in Figure 18 but analogous to that shown in Figure 5 with respect to sliding element 370) which enables the sliding element 670 to be mounted on the slider shaft 680. A connection (not shown) may also be provided at the proximal end 670e for connecting to a control wire as described above with reference to Figures 3 to 5.

The sliding element 670 has portions 670f (only one portion can be seen in Figure 18) formed along it length extending from near the proximal end 670e towards the distal end 670d and then extending into respective connecting elements 675f, 675g as shown. Connecting elements 675f, 675g include respective end portions 675h, 675i which provide flat engagement surfaces 675j, 675k for engaging with respective engagement surfaces 635j, 635k provided on the OTFC 635 (see Figure 16).

The end portions 675h, 675i are sprung inwards so that, when the OTFC 635 is inserted into the handle or housing 610 over the fixed shaft 630 extending through the distal end 670d of the sliding element 670, the proximal end 635b of the OTFC 635 forms a connection portion (described in more detail with reference to Figures 19 and 20 below) having an end surface 635d which engages sloping faces 675l, 675m of end portions 675h, 675i to push the end portions 675h, 675i apart until the engagement surfaces 635j, 635k of the OTFC 635 engage with respective engagement surfaces 675j, 675k of the end portions. Once the respective engagement surfaces are engaged, the OTFC 635 is held securely within the sliding element 670 mounted within the handle or housing 610.

Although only two end portions 675h, 675i are described with reference to Figure 18, it will readily be appreciated that any suitable number of end portions can be implemented to provide a secure engagement with the proximal end 635b of the OTFC 635.

It will also readily be appreciated that the one-touch fixed connection may be replaced by any other suitable fixing connection element which is attached to the hollow shaft.

Turning now to Figure 19, a hollow shaft (or OTFC) assembly is shown which comprises an OTFC 735 connected to a hollow shaft 730 at its distal end 735a. A removable cover (not shown) may be provided for the hollow shaft 730 prior to use. The OTFC 735 has a body portion 735c with a connection portion 735d at its proximal end 735b configured for connecting to a sliding element 670 described above with reference to Figure 18. The connection portion 735d comprises an end surface 735e having sloping faces 735l, 735m which engage sloping faces 675l, 675m of the sliding element 670 as described above with reference to Figure 18.

In addition, an indent 735f is provided adjacent the connection portion 735d which define engagement surfaces 735j, 735k which engage with respective engagement surfaces 675j, 675k of the sliding element 670 as described above. Once the end portions 675h, 675i pass over the end surface 735d along sloping faces 735l, 735m and into the indent 735f, the engagement surfaces 735j, 735k engage with respective engagement surfaces 675j, 675k of the sliding element 670 to hold the hollow shaft or OTFC assembly in place within the handle or housing 610 with the hollow shaft 730 located over the fixed shaft 620 (as described above with respect to Figures 16 and 17).

The body portion 735c of the OTFC 735 comprises a generally rounded body portion 735g with a flat surface 735h formed therein. The flat surface 735h provides an indication to a user as to the correct orientation for the insertion of the proximal end 735b into the sliding element 670 located within the handle or housing 610.

In the embodiment shown in Figure 19, the hollow shaft 730 has a curved portion 730c provided near its distal end 730a with a bevelled tip 730d at the distal end 730a. In use, the bevelled tip 730d is to be orientated such that the bevel faces upwards. The upwardly orientation of the bevelled tip 730d is aligned with the flat surface 735h of the OTFC 735 so that the hollow shaft or OTFC assembly can correctly be inserted into the handle or housing 610. As described above, the shunt or implant (not shown) is located at the distal end 730a of the hollow shaft 730 but back from the bevelled tip 730d so that it is fully within the hollow shaft 730.

Although not shown in Figure 19, it will be readily understood that the hollow shaft 730 extends through the body portion 735g and terminates substantially at the proximal end of the OTFC 735 so that the fixed shaft 620 (Figures 16 and 17) can be inserted therein as described above. In addition, the hollow shaft 730 is configured to retain a shunt or implant at its distal end 730a (also not shown).

Figure 20 illustrates another hollow shaft or OTFC assembly which comprises an OTFC 835 connected to a hollow shaft 830 at its distal end 835a. A removable cover (not shown) may be provided for the hollow shaft 830 prior to use. The OTFC 835 has a body portion 835c with a connection portion 835d at its proximal end 835b configured for connecting to a sliding element 670 described above with reference to Figure 18. The connection portion 835d comprises an end surface 835e having sloping faces 835l, 835m which engage sloping faces 675l, 675m of the sliding element 670 as described above with reference to Figure 18.

In addition, an indent 835f is provided adjacent the connection portion 835d which define engagement surfaces 835j, 835k which engage with respective engagement surfaces 675j, 675k of the sliding element 670 as described above. Once the end portions 675h, 675i pass over the end surface 835d along sloping faces 835l, 835m and into the indent 835f, the engagement surfaces 835j, 835k engage with respective engagement surfaces 675j, 675k of the sliding element 670 to hold the hollow shaft or OTFC assembly in place within the handle or housing 610 with the hollow shaft 830 located over the fixed shaft 620 (as described above with respect to Figures 16 and 17).

The body portion 835c of the OTFC 835 comprises a generally rounded body portion 835g with a flat surface 835h formed therein. The flat surface 835h provides an indication to a user as to the correct orientation for the insertion of the proximal end 835b into the sliding element 670 located within the handle or housing 610.

In the embodiment shown in Figure 20, the hollow shaft 830 is substantially straight with a bevelled tip 830d at the distal end 830a. In use, the bevelled tip 830d is to be orientated such that the bevel faces upwards. The upwardly orientation of the bevelled tip 830d is aligned with the flat surface 835h of the OTFC 835 so that the hollow shaft or OTFC assembly can correctly be inserted into the handle or housing 610. As described above, the shunt or implant (not shown) is located within the distal end 830a of the hollow shaft 830 so that it is fully within the hollow shaft 830.

Although not shown in Figure 20, it will be readily understood that the hollow shaft 830 extends through the body portion 835g and terminates substantially at the proximal end of the OTFC 835 so that the fixed shaft 620 (Figures 16 and 17) can be inserted therein as described above.

In another embodiment, the tip of the hollow shaft is flexible and bends to match scleral curvature at the iridocorneal angle so that the shunt or implant is positioned between the scleral spur and the ciliary body in the suprachoroidal space with a portion thereof extending into the anterior chamber to provide a drainage path to reduce IOP.

In the embodiments described with reference to Figures 16 to 20 above, a user (typically a surgeon) is provided with a handle 610 (Figure 16) with the fixed shaft 620 extending through the elongated aperture 690 at its distal end 610a. The handle 610 is preferably provided in a sterile package. The user is also provided with a hollow shaft or OTFC assembly (as described with reference to Figures 16, 19 and 20). The hollow shaft or OTFC assembly is also provided in a sterile package (with or without a removable cover) which may contain saline to prevent the shunt or implant from drying out during storage. Once the two sterile packages are opened, the hollow shaft or OTFC assembly is mounted in the handle or housing 610 by aligning the flat surface of the OTFC body with the handle 610 correctly before inserting the hollow shaft or OTFC assembly into the aperture 690 until the proximal end of the OTFC connects with the sliding element 670 within the handle or housing 610. The handle or housing 610 is also connected to an actuator and the system is then ready for use. It will readily be appreciated that the system may be supplied as a single item, that is, in a single package, with the hollow shaft or OTFC assembly already mounted to the handle or housing. In this case, the single package will be sterilised and may or may not comprise a removable cover as described above.

After use, the handle together with the hollow shaft or OTFC assembly (now without the shunt or implant) is disconnected from the actuator and discarded as these elements of the system are single-use. The actuator may be re-used after sterilisation, or even discarded. However, it will readily be appreciated that the handle may also be re-useable after appropriate sterilisation once the hollow shaft or OTFC assembly has been disconnected.

As an alternative or as an addition, other types of coding can be provided on the OTFC to ensure that the hollow shaft or OTFC assembly is correctly inserted into the handle or housing 610 prior to use.

In each of the embodiments described above, the hollow shaft comprises an atraumatic tip which mimimises trauma to the tissue into which it inserted and through which it passes.

As described above, the sliding element is operated by an actuating mechanism connected to the handle or housing by a wire. However, it will readily be appreciated that other ways of operating the slider may be possible. For example, a wireless link may be provided between an actuator and the handle or housing over which appropriate control signals can be sent to a servo-mechanism which retracts the sliding element with the hollow shaft leaving the shunt or implant in place between the anterior chamber and the suprachoroidal space if the suprachoroidal space is the intended implantation site.

Although the present invention has been described for a device or system for implanting a shunt or implant into the suprachoroidal space, other implantation sites also possible, for example, the subconjunctival space and the intra-scleral space. Ideally, the hollow shaft is configured to be flexible so as to match with the curvature of the ocular tissues into which the implant is intended to be placed.

It will readily be appreciated that the present invention is not limited to the embodiments described above, and that alternatives are possible.

## Claims

1. An implantation device for implanting an implant into a posterior space in an eye, the device comprising:
a housing;
a fixed shaft having a proximal end and a distal end, the proximal end being mounted within the housing and fixed with respect thereto, the distal end thereof extending from the housing;
a delivery mechanism comprising a sliding element mounted on the fixed shaft and at least partially within the housing, the sliding element being configured to be moved between a first position and a second position with respect to the housing and to at least the fixed shaft; and
a hollow shaft having a distal end and a proximal end and being configured to be mounted over the distal end of the fixed shaft and to be connected to the sliding element at its proximal end, the hollow shaft being configured to retain an implant within a portion thereof at its distal end, the hollow shaft being configured to be retracted over the fixed shaft by movement of the sliding element from its first to second positions to release the implant from within distal end of the hollow shaft;
wherein the delivery mechanism further comprises a slider shaft mounted within the housing and configured to be parallel to the fixed shaft, the sliding element being mounted on both the slider shaft and the fixed shaft.

2. A device according to claim 1, wherein the proximal end of the hollow shaft is mounted to a fixing element, the fixing element being configured for insertion into the housing over the fixed shaft and for engagement with the sliding element within the housing.

3. A device according to claim 2, wherein the fixing element comprises a connection portion configured to engage with a sliding element connection portion.

4. A device according to claim 3, wherein the connection portion of the fixing element comprises an indent providing at least two engagement surfaces, and, the sliding element connection portion comprises at least two end portions configured to be pushed apart by the insertion of the fixing element, the at least two end portions being configured to provide engagement surfaces which engage with the engagement surfaces of the fixing element.

5. A device according to any one of claims 1 to 4, wherein:
- the fixing element comprises a body portion having a surface thereof configured to indicate a correct orientation for insertion of the fixing element into the housing; and/or
- the fixing element comprises a one-touch fixing connection element.

6. A device according to any one of claims 1 to 5, wherein the hollow shaft has an internal profile which is configured to match substantially the external profile of the fixed shaft.

7. A device according to any one of claims 1 to 6, wherein the hollow shaft comprises a substantially transparent plastics material, the substantially transparent plastics material comprising one of: a thermosetting plastics material and a thermoplastics material.

8. A device according to any one of claims 1 to 6, wherein the hollow shaft comprises a biocompatible metal material.

9. A device according to any one of claims 1 to 8, wherein the hollow shaft comprises a bevelled tip.

10. A device according to any one of claims 1 to 9, wherein the distal end of the hollow shaft is configured to be flexible to match curvature of ocular tissues of the eye where the implant is to be implanted.

11. A device according to any one of claims 1 to 10, wherein the hollow shaft comprises at least one marker indicating an insertion depth for the posterior space with respect to an anterior chamber of the eye.

12. A device according to any one of claims 1 to 11, further comprising a light source configured to provide light for visual contrast between the implant and the posterior space of the eye;
- optionally wherein the light source comprises at least one light-emitting source configured to emit at least one colour of light; and
- optionally further comprising an optical waveguide connected to the light source and configured for directing light emitted by the light source to the distal end of the hollow shaft; optionally wherein the optical waveguide is formed on one of: at least a part of the

13. A device according to any one of claims 1 to 12, further comprising a button mounted on the housing and being configured to be moved with respect to the housing, the button being connected to the delivery mechanism and is configured to move the sliding element between its first and second positions when moved with respect to the housing.

14. An ocular implant system configured for implanting an implant into a posterior space in an eye, the ocular system comprising:
an implant configured for being implanted in the posterior space in the eye; and
an implantation device according to any one of claims 1 to 13, the implant being located in the distal end of the hollow shaft.

15. An ocular implant system according to claim 14, further comprising an actuating mechanism configured to be flexibly connected to the implantation device and being configured to move the sliding element of the delivery mechanism between its first and second positions;
- optionally wherein the actuating mechanism comprises a pneumatic plunger device configured to be connected to an operating mechanism, the operating mechanism being connected to the implantation device and comprising an inner portion and an outer portion at least partially surrounding the inner portion, the inner and outer portions being configured to be moveable relative to one another; and
- optionally wherein the implant comprises at least one marker configured to indicate an insertion depth of the implant within the posterior space with respect to the anterior chamber and the eye;
optionally wherein the marker comprises a colour chosen to provide contrast within the anterior chamber.
